# EUROPEAN PATENT SPECIFICATION

(11) **EP 2 779 905 B1**
(45) Date of publication and mention of the grant of the patent: **18.01.2017**
(21) Application number: 12857503.2
(22) Date of filing: 16.11.2012
(51) Int. Cl.: A61B 6/04, A61F 5/058, A61F 5/37, A61G 13/12

(54) **FIXTURE FOR IMMOBILIZING AN ARM OF A PATIENT**
VORRICHTUNG ZUR IMMOBILISIERUNG EINES ARMS EINES PATIENTEN
DISPOSITIF D'IMMOBILISATION D'UN BRAS D'UN PATIENT

(30) Priority: 16.11.2011 SE 1100856
(43) Date of publication of application: 24.09.2014
(73) Proprietor: All of it Scandinavia AB, 237 35 Bjärred (SE)
(72) Inventor: EKDAHL, Per, S-226 50 Lund (SE); BERGENUDD, Hampus, S-237 35 Bjärred (SE)
(74) Representative: Asketorp, Göran
(86) International application number: PCT/SE2012/000188
(87) International publication number: WO 2013/089608

(56) References cited:
- US-A- 2 693 794
- US-A- 3 256 880
- US-A- 3 540 719
- US-A- 3 776 225
- US-A- 5 623 951
- US-A- 5 845 643
- US-A- 6 045 520
- US-A1- 2005 052 066
- US-A1- 2010 049 110
- US-A1- 2011 155 148

## Description

### FIELD OF INVENTION

The present invention relates to a fixture for an arm of a patient. The fixture is especially intended to be used during X-ray investigation and evaluation and during possible balloon expansion of coronary vessels of the heart.

### BACKGROUND

Heart deceases are the most common cause of death in the Western World.

The most common heart decease is coronary artery obstruction. The coronary arteries provide oxygenated blood to the heart muscle itself. Obstructions result in less blood flow, often resulting in angina pectoris, which is chest pain due to ischemia, i.e. lack of blood supply, thus a lack of oxygen supply of the heart muscle, generally due to obstruction or spasm of the coronary arteries.

In order to confirm coronary vessel decease, the coronary vessels are exposed to X-ray investigation, which today is considered to be the "Golden Standard". If obstructions or constrictions are found, such constrictions may be treated by balloon expansion or surgery, such as by-pass surgery, or by other methods. Obstructions, such as blood clots, may be dissolved by introduction of suitable agents.

The X-ray investigation and evaluation and other interventions may be performed by inserting a catheter extending to the heart via an artery, such as arteria femoralis or arteria radialis.

When the investigation takes place via the radial artery, it may be convenient for the surgeon to use the right arm of the patient lying in a supine position, since the surgeon almost always is placed at the right side of the patient. However, if the patient has been exposed to a previous by-pass surgery, an implant is often arranged in a vessel adjacent the clavicle. The right arm cannot be used for such patients but the left arm radial artery has to be used. Also of other reasons, it may be advantageous to use the left arm instead of the right arm of the patient.

When the left arm is used and the surgeon is arranged at the right side of the patient, the arm should be placed on the abdomen of the patient approximately at the middle of the patient. If the left arm is placed in this position, the palm of the hand is normally faced downward towards the abdomen and the radial artery at the wrist is not available. The arm needs to be rotated at least about 90 degrees around its symmetry axis and the wrist should be turned backward to expose the inner wrist portion, where the radial artery is most easily available. There is a need for a fixture for obtaining such a position.

Document US 2693794 discloses a portable brace for restraining movement of a person's arm during medical or surgical treatment, such as during intravenous feeding through an arm vein. The brace comprises a main restraint member of a rigid material substantially semi-cylindrical in form and of a diameter sufficient to receive and to hold at least the back and side portions of the arm of a patient, leaving the inner surfaces of the wrist, forearm and upper arm exposed. The restraint member has a length to extend from the hand to a point above the elbow. The brace further comprises a transverse cylindrical handle, at the front end of the restraint member and positioned to be grasped by the hand of the patient whose arm is held in said restraint member. The restraint member has a substantially U-shaped cutout in its underside to provide space for the knuckles and back of the patients hand. The brace further comprises a forward restraining strap secured at one end to one side of the restraint member in the vicinity of the wrist retaining portion, and arm restraining strap secured at one end to one side of the restraint member near the upper end of the restraint member, and means for detachably securing the other ends of said straps to said restraint member after the straps have been passed across the arm of a patient.

Document US 3256880 discloses a main or forearm rest which may comprise a relatively thin, straight element adapted to extend substantially from the wrist to the elbow. It may be formed with curved side walls generally conforming to the shape of the forearm. The forearm rest may be provided with resilient fastening means at each end thereof for securing it to the forearm of a patient in a manner to immobilize the forearm. The forward end of the forearm element may include spaced parallel slots for receiving the bifurcated end of a hand rest that includes a planar surface adjacent the bifurcated end and an angularly deflected surface at the end of the hand rest opposite its bifurcated end. Flexible strap means may also be connected to the hand rest for immobilizing the patient's hand, and the construction is such that it can be attached to the forearm rest with its concave surface formed by the angularly deflected portion facing either upwardly or downwardly to accommodate the fingers in two positions of the hand.

Document US 3540719 discloses an adjustable forearm rest apparatus of articulated type with hand holding means which is extensible or retractable and angularly positionable on a support bracket. The bracket may be mounted selectively on a stand of adjustable type or attached to a hospital bed and the apparatus includes an upper arm support and means for holding instruments and devices used in cardiovascular studies, etc.

Document US 3776225 discloses an arm splint for supporting and maintaining a patient's forearm immobile when desired, such as, for example, when receiving an intravenous injection. The splint comprises an elongated strip terminating below but adjacent the elbow at one end and terminating adjacent the middle phlangeal joint of the fingers at the other end and having upwardly extending side walls at the opposite ends to prevent undesirable lateral movement of the arm and fingers. Means for securing the splint to the arm of the user are provided, said means being adjustable lengthwise with respect to the splint on a sliding track. The splint further comprises a second strip that may be adjustably and removably secured to the elbow end of the first strip when it is desired to maintain the user's arm completely straight.

However, if conventional fixtures for arms, and especially the elbow portion of the arm, is used, such fixtures normally comprises straps extending around the arm for attachment of the fixture to the arm. Such straps have a tendency to strangle the underlying artery and make difficult and even impossible to insert a catheter via the radial artery and further to the heart. Thus, there is a need for a new type of fixtures, that does not strangle the underlying artery.

### SUMMARY OF THE INVENTION

Accordingly, an object of the present invention is to mitigate, alleviate or eliminate one or more of the above-identified deficiencies and disadvantages singly or in any combination.

In an aspect, there is provided a fixture for being attached to an arm of a patient, comprising a first portion, a second portion and a third portion, the first portion being intended to be arranged adjacent an elbow at the inner side of the elbow and extending from the inner side of the elbow up along an upper arm portion and being arranged integral with the second portion at a first angle, which is between 110 degrees and 170 degrees; the second portion having a length which is smaller than or equal to a forearm and supporting the forearm; the third portion being integral with or connected to the second portion at a second angle, which is between 190 degrees and 250 degrees. The second portion is divided in two parts interconnected by a semi-bracelet, whereby a first part of the second portion is arranged integral with said first portion at said first angle and a second part of the second portion is integral with or connected to the third portion at said second angle; and wherein the semi-bracelet extends over about 180 degrees around a symmetry axis of the arm, whereby the first part is arranged at the inner side of the arm and the second part is arranged at the outer side of the arm.

The fixture may be attached to the arm of the patient by several flexible straps. The length of the second portion may be adjustable. At least a member of the fixture may be sterilized.

In an embodiment, the fixture may further comprise a fastening means for fastening the fixture in relation to the body of the patient, such as a fastening means adjacent the third portion intended for attachment to a belt.

### BRIEF DESCRIPTION OF THE DRAWINGS

Further objects, features and advantages of the invention will become apparent from the following detailed description of embodiments of the invention with reference to the drawings, in which:
Fig. 1 is a schematic view of a patient lying in a supine position with his left hand in a resting position at his abdomen.
Fig. 2 is a schematic view similar to Fig. 2 in which the desired position of the hand and the wrist is shown.
Fig. 3 is a schematic view similar to Fig. 1 in which a prior art fixture is attached to an arm.
Fig. 4 is a side view of the fixture according to Fig. 3.
Fig. 5 is a schematic view similar to Fig. 1 in which a first embodiment of a fixture is attached to the arm.
Fig. 6 is a side view of the first embodiment of the fixture.
Fig. 7 is a schematic view similar to Fig. 5 in which an adjustment strap is arranged.
Fig. 8 is a side view of a second embodiment of the fixture.

### DETAILED DESCRIPTION OF EMBODIMENTS

Below, several embodiments of the invention will be described. These embodiments are described in illustrating purpose in order to enable a skilled person to carry out the invention and to disclose the best mode. However, such embodiments do not limit the scope of the invention. Moreover, certain combinations of features are shown and discussed. However, other combinations of the different features are possible within the scope of the invention.

When a catheter should be used for X-ray investigation and possible balloon expansion, the most common way to insert such a catheter has up to now been insertion into the femoral artery, since it is sufficiently large and extends directly to the aortic arc and subsequently to the coronary arteries. However, since the femoral artery is large, there is also a concomitant risk of blood leakage and complications.

As an alternative, the use of the radial artery in the arm has been suggested. The surgeon is normally placed at the right side of the patient and has a heavy lead apron in order to shield the surgeon from X-rays. Thus, it may be convenient for the surgeon to use the right arm and difficult and cumbersome to use the left arm of the patient. However, the use of the left arm results in that the catheters may be arranged in a more convenient position. In addition, the use of the right arm may be impossible due to previous interventions. Thus, the embodiments shown below are intended to be used at the left arm of the patient when the surgeon is placed on the right side of the patient. However, the different embodiments may also be used at the right arm of the patient.

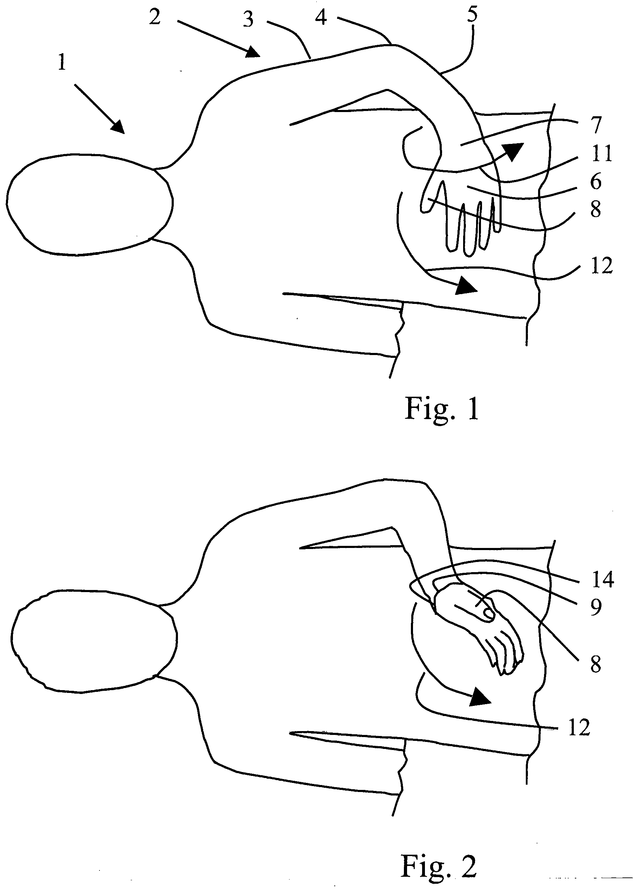

Fig. 1 shows a patient 1 arranged in a supine position with his left arm 2 resting on the abdomen with his hand 6 approximately at the navel. The arm 2 has an upper arm portion 3 and a forearm 5 at each side of the elbow 4. The hand 6 is attached to the forearm 5 via the wrist 7.

The palm of the hand 6 is facing downward towards the abdomen when the patient is in the supine position, as appears from Fig. 1, and the thumb 8 is directed towards the face of the patient. Thus, the palm of the hand and the inner side of the wrist 7 are facing downwards, towards the abdomen and the radial artery is not conveniently available. The palm of the hand is approximately horizontal.

The expressions "inner" and "outer" are intended to mean positions at the normal arm position. Thus, the inner side of the arm is the side of the arm normally facing the abdomen, for example in the position shown in Fig. 1. The outer side of the arm is the portion of the arm facing away from the abdomen. The inner side of the hand, i.e. the palm, is facing the abdomen in the position shown in Fig. 1.

As shown by arrow 11 in Fig. 1, the hand 6 and the wrist 7 may be rotated or twisted about 90 degrees around the symmetry axis of the arm in order to expose the radial artery 9, shown in Fig. 2, at the inner side of the wrist. In this position, the palm surface of the hand forms approximately a right angle with the abdomen and the thumb faces upwards, i.e. the palm of the hand is oriented in a substantially vertical direction when the patient is arranged in the supine position shown in Figs. 1 and 2. In this twisted position of the arm and the wrist, the radial artery 9 of the left arm is conveniently available for insertion of a catheter in the artery as shown in Fig. 2.

In addition, the wrist should be turned so as to open the angle of the wrist, as shown by arrow 12. The expression "opening" the angle of the wrist is intended to mean that the angle between the forearm 5 and the palm of the hand 6 becomes larger than 180 degrees. In this way the radial artery 9 will be conveniently available.

As an alternative to the radial artery, the surgeon may use an ulnar artery 14 arranged at the position shown in Fig. 2. The ulnar artery is also conveniently available.

It is noted that in Fig. 2 the elbow angle and the wrist angle are arranged in substantially the same plane, but in different directions.

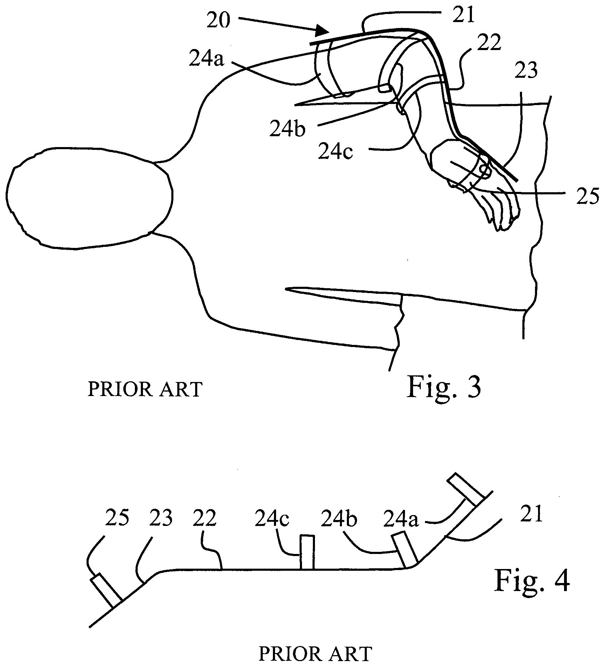

Thus, a fixture with the shape shown in Fig. 3 may be used for arranging the arm in the desired position as indicated above.

The fixture shown in Fig. 3 (prior art) comprises three interconnected portions, viz. a first portion 21, a second portion 22 and a third portion 23.

The first portion 21 is intended to be arranged at the outside of the upper arm as shown and extending to the elbow.

The second portion 22 is connected to the first portion 21 at a first angle, which may be about 150 degrees. The second portion 22 has a length corresponding to the forearm 4 and supports the forearm. The second portion 22 may have an adjustable length in order to suit patients having different arm lengths.

The third portion 23 is connected to the second portion 22 at a second angle, which is opposite to the first angle and may be of the same size but in the opposite direction. Thus, the second angle is about 210 degrees. The third portion 23 extends along the hand back.

The fixture 20 may be connected to the patient by several straps at suitable positions, as shown in Fig. 3. Thus, first, second and third straps 24a, 24b, 24c may be arranged in a position at the middle of the upper arm 3. A hand strap 25 may be arranged adjacent the hand. The hand strap 25 may be arranged to include the thumb as shown in Fig. 3 or may be arranged below the thumb. The fixture 20 is shown in side view in Fig. 4.

However, the three straps 24a, 24b, 24c will be arranged at the inner side of the upper arm 3 and forearm 5 as shown in Fig. 3. These straps have a tendency to squeeze the artery underlying the straps. The radial artery 9 and also the ulnar artery 14 extend between the skin of the inner portion of the arm and the bone of the arm.

The forces of the straps are distributed over a relatively small surface, which means that the straps penetrate into the underlying skin, tissue and muscles. Especially the second strap 24b may penetrate deeply into the underlying tissue at the inner portion of the elbow and influence upon the arteries there below, but also the first strap 24a and the second strap 24c will provide a strangling action, especially when the fixture is exposed to tension.

Thus, the artery is partially strangled by the straps, which means that a catheter that should be inserted in the artery will have difficulties in passing said strangled areas of the artery. There is also a risk that the artery wall will be scraped or damaged when the catheter is inserted or manipulated.

Thus, there is a need for a fixture, which does not strangle the artery.

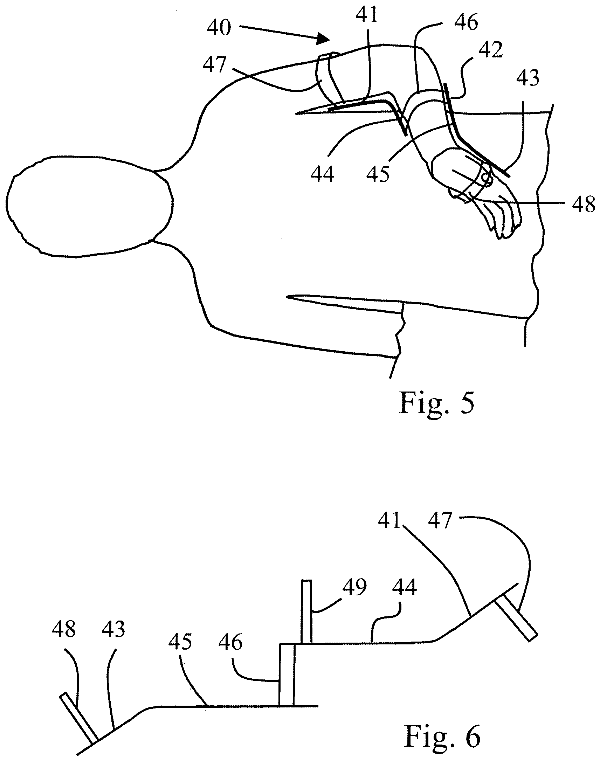

In a first embodiment of the fixture, the above-mentioned problem may be counteracted by providing a fixture, wherein the first portion 21 and a part of the second portion are arranged at the inner side of the elbow, as shown in Fig. 5. In this manner, the forces from the fixture towards the inner surface of the arm will be distributed over the entire surface of the fixture toward the arm and the skin, and the strangling action of the straps will be exerted at the outer side of the arm, where the strangling action does not influence upon the arteries.

The fixture 40 according to the first embodiment comprises three interconnected portions, viz. a first portion 41, a second portion 42 and a third portion 43.

The first portion 41 is intended to be arranged at the inside of the elbow as shown in Fig. 5 and extending along the upper arm portion 3. The first portion 41 is connected to the second portion 42 at a first angle, which may be about 150 degrees. The third portion 43 is connected to the second portion 42 at a second angle, which may be about 210 degrees.

The second portion 42 is divided into two parts, a first part 44 connected to the first portion 41, and a second part 45 connected to the third portion 43. The first part 44 and the second part 45 are interconnected by a half bracelet 46, which encircles half or about 180 degrees of the forearm 4, such as at the outer side of the forearm 4, as shown in Fig. 6. Thus, the half bracelet is called a semi-bracelet below. The semi-bracelet is relatively stiff and is fixedly connected to both the first part 44 and the second part 45. Thus, the fixture 40 forms an integral, relatively rigid, single unit.

By means of the bracelet, the first part 46 and the second part 45 are arranged in parallel but off-set by a distance. The distance substantially corresponds to the diameter of the arm. By this arrangement, the first part 46 may be arranged at the inner side of the arm while the second part 45 is arranged at the outer side of the arm.

However, the attachment of the second part 45 to the bracelet 46 may be adjustable in the longitudinal direction in order to accommodate arms of different lengths.

Alternatively, the first part 44, or both the first and the second part are adjustably connected to the bracelet for adjustment purpose.

The first, second and third portions may have a transversal dimension so that it encircles a portion of the arm, such as about one sixth (60 degrees) of the periphery of the arm. The fixture portion facing the arm may be provided with soft material, so that the forces from the fixture to the arm are evenly distributed. The width of the fixture perpendicular to or transversal to the arm symmetry axis may be about 4 to 12 cm, such as about 6 to 10 cm, for example 8 cm.

The surface of the fixture facing the arm may be provided with a shallow recess extending in the longitudinal direction, so that the arm can rest conveniently in the recess.

The fixture 40 may be connected to the patient by several straps at suitable positions, as shown in Fig. 5. Thus, a first strap 47 may be arranged at a position at the middle of the upper arm 3. A second strap 48 may be arranged adjacent the hand. A third strap (not shown) may be arranged adjacent the semi-bracelet and encircling the entire forearm outside the fixture.

The fixture may be arranged at the patient when the arm is resting along the side of the patient. Alternatively, the arm is brought in the desired position and the fixture is arranged at the arm.

The fixture according to the first embodiment may follow the curvature of the body in a convenient manner without interference with the body.

The fixture according to the first embodiment is shown in a side view in Fig. 6. The third strap 49 is shown slightly beside the semi-bracelet 46, but it may be arranged in any desired position in which it attaches the forearm to the fixture.

It may be desired to move the arm, after being arranged in the fixture, to a position in which the arm and the wrist is more conveniently available. For this purpose, a strap 50 is attached to the first portion 41 and/or the second portion 42, for example at the first part 44 adjacent the semi-bracelet 46. The strap 50 extends over the body to the right side of the patient, wherein the surgeon may pull the strap 50 in order to move the arm towards himself.

The strap 50 may be attached to the bed of the patient in order not to extend outside the bed.

The strap 50 may be arranged to exert a pulling force to the arm in order to arrange the arm in a desired position and counteract a tendency of the arm to fall back to the left side of the body.

Such a pulling force will at the same time relieve the inner surface of the arm from any pressure from the fixture, thereby further preventing any influence upon the underlying artery.

The strap 50 is attached to the fixture in a position as close to the body as possible, in order not to rotate the arm in a non-desired direction, in which the wrist becomes less available.

The strap 50 may in addition extend in the other direction, as shown by broken lines 51, whereupon a nurse arranged at the left side of the patient may interact with the strap, for example to stretch the strap.

The strap 50 may be arranged to pass below the bed to the right side of the bed, so that the surgeon can move the fixture and the arm in both directions. In this arrangement, a nurse at the left side of the patient may interact with the strap portion 51 in order to move the arm towards the surgeon by pulling in the portion extending below the bed.

The strap 50 may be attached to the fixture at any position, which makes it operate as desired. However, if the strap is attached close to the third portion or at the third portion 43, the strap 50 may interact with the catheter to be inserted in the artery of the patient, and, thus, such a position is sometimes non-desired. In addition, the strap may be contaminated with blood.

Although there is presently no requirement for sterilization of the fixture, there may be reasons to keep the fixture as non-contaminated as possible, in order to avoid the introduction of germs or bacteria close to the insertion point of the catheter.

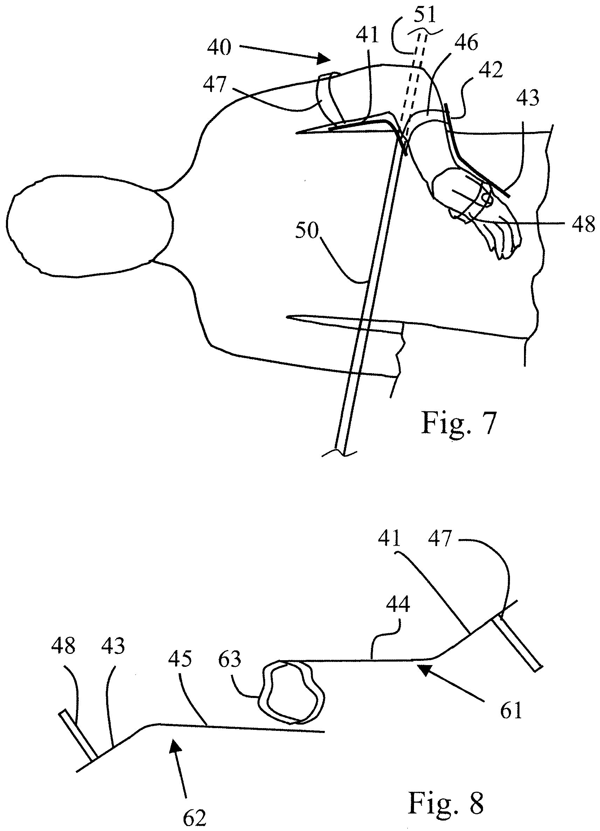

In a second embodiment shown in Fig. 8, the fixture is made up of two separate members, a first member 61 comprising the first portion 41, the first part 44 of the second portion 42 and the semi-bracelet. A second member 62 comprises the second part 45 of the second portion 42 and the third portion 43. The second member is attached to the first member by means of fasteners of any type, such as snap fasteners, a Velcro fastener or a fastener similar to a fastener shown below with reference to Fig. 11.

The division of the fixture in two members 61 and 62 makes it possible to sterilize the second member 62, which is close to the insertion point of the catheter. Thus, the second member 62 may be disposable. This is also an advantage in the case wherein blood has contaminated the second member.

The first member 61 may be reusable and may be made of a material, which may be cleaned in a suitable manner.

It is noted that the entire fixture is made of a material, which is transparent to X-rays, which excludes metals. Thus, the fixture may be made of a plastics material, such as reinforced epoxy plastics material. The second member 62 is made of a material, which may be sterilized.

The fixture may comprise small details of a non-transparent material, such as screws or rivets or metal, but such details should be arranged in areas so that they do not interfere with the blood vessels to be examined or used.

In the first and the second embodiment, the semi-bracelet does not need to be rigid. The semi-bracelet may be made of a flexible material that has a certain spring action so that the bracelet can be arranged at forearms of different sizes.

In an alternative design shown in Fig. 8, the semi-bracelet 46 is completely replaced by a strap 63, which encircles the forearm approximately at the middle thereof. The strap 63 is flexible but is non-elastic. The strap 63 is attached to the forearm sufficiently rigidly for keeping the forearm in the desired rotational position, but does not strangle the underlying artery, because the inner side of the part 44 has a sufficiently large surface to spread the forces.

The second member 62 is attached to the strap 49 by quick release fasteners. The second member 62 may be attached to the strap 63 at a suitable longitudinal position so that the combined first part 44 and second part 45 corresponds to the length of the forearm.

In one arrangement, the second part 45 is only inserted between the strap 63 and the forearm after the upper portion 44 has been arranged in place.

In certain applications, wherein the requirement on the fixture is less stringent, it may be sufficient to use only the first member 61. In this case, the forearm is fixed in the desired rotational position by the strap 63, which is firmly arranged around the forearm.

The strap 63 may be provided with friction material at the surface facing the arm, in order to hold the forearm in the desired rotational position. The friction material may be rubber or an elastomer. Any of the straps may be provided with such friction material.

The straps may be made of a flexible material and having a length corresponding to the diameter of the respective arm portion. The end of the strap may be provided with a fastening means, such as a Velcro strap. The fixture may be provided with a Velcro fastener at a suitable position in order to interact with the Velcro strap.

Alternatively, two straps may be arranged extending in opposite directions and ending with mating Velcro fasteners.

There may be arranged additional semi-bracelets in order so support the fixture in relation to the arm. The supporting semi-bracelet may extend over 180 degrees or over still smaller angle such as 120 degrees or 90 degrees. Thus, a supporting bracelet may be arranged at the middle of the second part 44 and encircling half the arm at the outer side thereof.

The fixture may be made of glass-fiber reinforced plastics material, such as an epoxy plastics material. Other suitable materials may be PVC and polyurethane. The material should be of the type, which is permitted to be used in medical applications. The material should be transparent to X-ray radiation.

The fixture may be covered by textile at the surfaces facing the arm or the body. The straps may also be covered by a soft material or a material having friction towards the arm or the skin, such as rubber or an elastomer.

Further alternative combinations of the different features shown in the different embodiments are possible within the scope of the invention.

The first angle may be from about 170 degrees to about 110 degrees. The second angle may be from about 190 degrees to about 250 degrees. In the third embodiment, the second angle may be from about 170 degrees to 110 degrees. The first and second angles may be adjustable.

The different embodiments have been described in an application wherein a catheter should be introduced into the radial or ulnar artery of the left arm. Such catheter may be used in heart investigations and intervention.

There are other medical methods when it may be desired to immobilize the left arm in the position shown, for example in emergency situations when fast and convenient access to the arm artery or vein is desired.

Another medical method may be the introduction of cold saline in the arm artery for cooling of the body as soon as possible after a brain injury, such as brain hemorrhage or stroke. About 500 to 1000 ml cold saline is introduced in the body as soon as possible to induce hypothermia.

A further medical method in which the fixture may be used is the localization and dissolution of clots or obstructions in other positions than the heart.

In the claims, the term "comprises/comprising" does not exclude the presence of other elements or steps. Furthermore, although individually listed, a plurality of means, elements or method steps may be implemented by e.g. a single unit. Additionally, although individual features may be included in different claims or embodiments, these may possibly advantageously be combined, and the inclusion in different claims does not imply that a combination of features is not feasible and/or advantageous. In addition, singular references do not exclude a plurality. The terms "a", "an", "first", "second" etc. do not preclude a plurality. Reference signs in the claims are provided merely as a clarifying example and shall not be construed as limiting the scope of the claims in any way.

Although the present invention has been described above with reference to specific embodiment and experiments, it is not intended to be limited to the specific form set forth herein. Rather, the invention is limited only by the accompanying claims and, other embodiments than those specified above are equally possible within the scope of these appended claims.

## Claims

1. A fixture for being attached to an arm of a patient, comprising a first portion (41), a second portion (42) and a third portion (43),
the first portion (41) being intended to be arranged adjacent an elbow at the inner side of the elbow and extending from the inner side of the elbow up along an upper arm portion (3) and being arranged integral with the second portion (42) at a first angle, which is between 110 degrees and 170 degrees;
the second portion (42) having a length which is smaller than or equal to a forearm (5) and supporting the forearm;
the third portion (43) being integral with or connected to the second portion (42) at a second angle, which is between 190 degrees and 250 degrees,
**characterized in that**
the second portion is divided in two parts (44, 45) interconnected by a semi-bracelet (46), whereby a first part (44) of the second portion (42) is arranged integral with said first portion (41) at said first angle and a second part (45) of the second portion (42) is integral with or connected to the third portion (43) at said second angle; and
wherein the semi-bracelet (46) extends over about 180 degrees around a symmetry axis of the arm, whereby the first part (44) is arranged at the inner side of the arm and the second part (45) is arranged at the outer side of the arm.

2. The fixture according to claim 1, wherein said fixture is attached to the arm of the patient by several flexible straps (47, 48).

3. The fixture according to claim 1 or 2, wherein the length of the second portion is adjustable.

4. The fixture according to any one of the previous claims, further comprising a fastening means for fastening the fixture in relation to the body of the patient, such as a fastening means adjacent the third portion intended for attachment to a belt (50).

5. The fixture according to any one of the previous claims, wherein at least a member of the fixture is made of a material that can be sterilized.

## Patentansprüche

1. Vorrichtung zur Anbringung an einem Arm eines Patienten, umfassend einen ersten Abschnitt (41), einen zweiten Abschnitt (42) und einen dritten Abschnitt (43),
wobei der erste Abschnitt (41) dafür vorgesehen ist, benachbart zu einem Ellenbogen an der Innenseite des Ellenbogens angeordnet zu werden und sich von der Innenseite des Ellenbogens nach oben entlang eines Oberarmbereichs (3) zu erstrecken und integral mit dem zweiten Abschnitt (42) in einem ersten Winkel angeordnet zu sein, welcher ein Winkel zwischen 110 und 170° ist;
wobei der zweite Abschnitt (42) eine Länge aufweist, die kleiner ist oder gleich einem Unterarm (5) und den Unterarm unterstützt;
wobei der dritte Abschnitt (43) integral ist mit oder verbunden ist mit dem zweiten Abschnitt (42) in einem zweiten Winkel, welcher ein Winkel zwischen 190° und 250° ist,
**dadurch gekennzeichnet, dass**
der zweite Abschnitt in zwei Bereiche (44,45) unterteilt ist, die durch ein Halb-Armband (46) miteinander verbunden sind, wobei ein erster Bereich (44) des zweiten Abschnitts (42) in dem ersten Winkel integral mit dem ersten Abschnitt (41) angeordnet ist, und wobei ein zweiter Bereich (45) des zweiten Abschnitts (42) in dem zweiten Winkel integral ist mit oder mit dem dritten Abschnitt (43) verbunden ist; und
wobei sich das Halb-Armband (46) über ungefähr 180° um eine Symmetrieachse des Arms herum erstreckt, wobei der erste Bereich (44) auf der Innenseite des Arms und der zweite Bereich (45) an der Außenseite des Arms angeordnet sind.

2. Vorrichtung nach Anspruch 1, wobei die Vorrichtung mithilfe von mehreren flexiblen Riemen (47,48) an dem Arm des Patienten angeordnet ist.

3. Vorrichtung nach Anspruch 1 oder 2, wobei die Länge des zweiten Abschnitts verstellbar ist.

4. Vorrichtung nach einem der vorhergehenden Ansprüche, weiter umfassend ein Befestigungsmittel zum Befestigen der Vorrichtung im Verhältnis zu dem Körper des Patienten, wie zum Beispiel ein Befestigungsmittel, benachbart zu dem dritten Abschnitt, das zur Anbringung an einen Gürtel (50) vorgesehen ist.

5. Vorrichtung nach einem der vorhergehenden Ansprüche, wobei wenigstens ein eine Komponente der Vorrichtung aus einem Material hergestellt ist, das sterilisiert werden kann.

## Revendications

1. Dispositif destiné à être attaché à un bras d'un patient, comprenant une première portion (41), une deuxième portion (42) et une troisième portion (43),
la première portion (41) étant destinée à être disposée adjacente à un coude sur le côté intérieur du coude et s'étendant du côté intérieur du coude vers le haut le long d'une portion de bras supérieure (3) et étant agencée d'un seul tenant avec la deuxième portion (42) selon un premier angle, qui est entre 110 degrés et 170 degrés ;
la deuxième portion (42) présentant une longueur qui est inférieure ou égale à un avant-bras (5) et soutenant l'avant-bras ;
la troisième portion (43) étant d'un seul tenant avec ou reliée à la deuxième portion (42) selon un deuxième angle, qui est entre 190 degrés et 250 degrés,
**caractérisé en ce que**
la deuxième portion est divisée en deux parties (44, 45) reliées entre elles par un demi-bracelet (46), moyennant quoi une première partie (44) de la deuxième portion (42) est agencée d'un seul tenant avec ladite première portion (41) selon ledit premier angle et une seconde partie (45) de la deuxième portion (42) est d'un seul tenant avec ou reliée à la troisième portion (43) selon ledit deuxième angle ; et
dans lequel le demi-bracelet (46) s'étend sur environ 180 degrés autour d'un axe de symétrie du bras, moyennant quoi la première partie (44) est disposée sur le côté intérieur du bras et la seconde partie (45) est disposée sur le côté extérieur du bras.

2. Dispositif selon la revendication 1, dans lequel ledit dispositif est attaché au bras du patient par plusieurs bandes flexibles (47, 48).

3. Dispositif selon la revendication 1 ou 2, dans lequel la longueur de la deuxième portion est réglable.

4. Dispositif selon l'une quelconque des revendications précédentes, comprenant en outre un moyen de fixation pour fixer le dispositif par rapport au corps du patient, tel qu'un moyen de fixation adjacent à la troisième portion destiné à être attaché à une ceinture (50).

5. Dispositif selon l'une quelconque des revendications précédentes, dans lequel au moins un élément du dispositif est fabriqué en un matériau qui peut être stérilisé.
